(19) Europäisches Patentamt
European Patent Office
Office européen des brevets

(11) **EP 1 413 319 B1**

(12) # EUROPEAN PATENT SPECIFICATION

(45) Date of publication and mention of the grant of the patent:
**05.07.2006 Bulletin 2006/27**

(51) Int Cl.:
***A61L 15/60*** *(2006.01)*

(21) Application number: **03004204.8**

(22) Date of filing: **25.02.2003**

(54) **Absorbent material and absorbent article**

Absorbierendes Material und absorbierender Artikel

Materiau absorbant et article absorbant

(84) Designated Contracting States:
**BE DE IT NL**

(30) Priority: **24.10.2002 JP 2002309545**

(43) Date of publication of application:
**28.04.2004 Bulletin 2004/18**

(73) Proprietor: **DAINIPPON INK AND CHEMICALS, INC.**
**Itabashi-ku**
**Tokyo (JP)**

(72) Inventors:
- **Tanaka, Hisakazu**
  **Izumiotsu-shi,**
  **Osaka (JP)**
- **Hasegawa, Yoshiki**
  **Nishinomiya-shi,**
  **Hyogo-ken (JP)**
- **Asada, Masahiko**
  **Sakura-shi,**
  **Chiba-ken (JP)**

(74) Representative: **Albrecht, Thomas et al**
**Kraus & Weisert,**
**Thomas-Wimmer-Ring 15**
**80539 München (DE)**

(56) References cited:

| | |
|---|---|
| **EP-A- 0 413 627** | **EP-A- 0 761 241** |
| **EP-A- 1 233 032** | **WO-A-95/22357** |

- **MASAYO OYA ET AL: 'mercury Intrusion Porosimetry' AMERICAN CERAMIC SOCIETY BULLETIN vol. 81, no. 3, March 2002,**
- **[Online] Mercury Intrusion Porosimetry - Acsion Retrieved from the Internet: <URL:http: //www.acsion.com/index.cfm>**
- **[Online] Mercury Penetration Porosimetry Retrieved from the Internet: <URL:http: //www.google.de/search?hl>**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 413 319 B1

## Description

BACKGROUND OF THE INVENTIION

Field of the Invention

The present invention relates to a novel and useful absorbent material and relates to an absorbent article.

Description of the Related Art

As articles for absorbing blood, such as panty liners and sanitary napkins, for example, there have hitherto been known those comprising a liquid permeable skin material made of a nonwoven fabric, a liquid impermeable leakproof material made of a polyethylene sheet or a polyethylene sheet laminate nonwoven fabric, and an absorbent core made of hydrophilic absorbent paper or cotton-like pulp, capable of physically absorbing and retaining blood, the absorbent core being interposed between the liquid permeable skin material and the absorbent core. With respect to the material of the absorbent core, it has been proposed to improve the absorption capacity for blood by using a water-absorbent resin in place of absorbent paper or pulp and to prevent leakage by retaining blood after absorption.

As this kind of the water-absorbent resin, for example, there have hitherto been known hydrolyzate of starch-acrylonitrile graft copolymer, carboxymethylcellulose crosslinked body, polyacrylate (salt) crosslinked body, acrylate (salt)-vinyl alcohol copolymer and polyethylene oxide crosslinked body. However, since these conventional water-absorbent resins were developed to absorb urine, not blood, aggregation between particles is enhanced by adsorption of protein, blood cell components and decomposition products of tissue in blood on the surface of a high water-absorbent resin and gel blocking is promoted, and thus blood absorption characteristics were drastically lowered.

As one means for solving this problem and improving the absorbency of the water-absorbent resin to blood, a modification method of increasing the surface area of the water-absorbent resin is known. As the method of preparing a water-absorbent resin agglomerate having large surface area, there is disclosed a technique of supplying an aqueous solution of a water-soluble polymerizable monomer containing a phosphoric ester type surfactant in a hydrophobic organic solvent containing a phosphoric ester type surfactant and effecting the reverse suspension polymerization (see, for example, Patent Document 1). However, since this method was developed to exclusively improve absorption characteristics for urine, absorption characteristics for blood were not yet improved.

Also, there is disclosed a porous absorbent material obtained by allowing a water-absorbent polymer containing a positive charge donor compound and a nonionic compound to absorb water to swell the water-absorbent polymer, and removing water while maintaining the swollen state by a freeze-drying method (see, for example, Patent Document 2). However, since the porous absorbent material is obtained by allowing the water-absorbent polymer to absorb water to swell the water-absorbent polymer and freeze-drying the swollen water-absorbent polymer, the porous absorbent material had a brittle structure and a porous structure is likely to be broken when pressure is applied.

Under these circumstances, there has been a great desire to develop an absorbent material having a sufficient structural strength wherein absorption characteristics are not drastically lowered by blood components.

(Patent Document 1)
Japanese Patent Application, First Publication No. 2001-2712A (page 2, claims, pages 4 and 5)
(Patent Document 2)
WO95/22357 (pages 22-25, pages 34-35, Example 1)

SUMMARY OF THE INVENTION

An object of the present invention is to provide an absorbent material having a sufficient structural strength and improved absorption characteristics for blood, and to provide an absorbent article.

The present inventors have intensively reserched to achieve the object described above and found that it is made possible to achieve a sufficient structural strength and to improve the wettability to blood by using an absorbent resin, which is composed of a specific resin and includes specific pores, and also absorption characteristics of blood can be improved, and thus the present invention has been completed.

The present invention provides an absorbent material comprising, as a main component, an absorbent resin which contains a polyacidic amino acid as a constituent component of the resin and has such a structure that primary particles are agglomerated, and also includes pores having a total pore volume as measured by a mercury penetration method (mercury intrusion porosimetry method) of 0.5 to 5.0 $cm^3/g$.

Also, the present invention provides an absorbent article comprising an absorbent core including an absorbent material and a fiber material, and a sheet-like material provided on both surfaces of the absorbent core, wherein absorbent

material is an absorbent material comprising, as a main component, an absorbent resin which contains a polyacidic amino acid as a constituent component of the resin and has such a structure that primary particles are agglomerated, and also includes pores having a total pore volume as measured by a mercury penetration method (mercury intrusion porosimetry method) of 0.5 to 5.0 $cm^3/g$.

BRIEF DESCRIPTION OF THE DRAWING

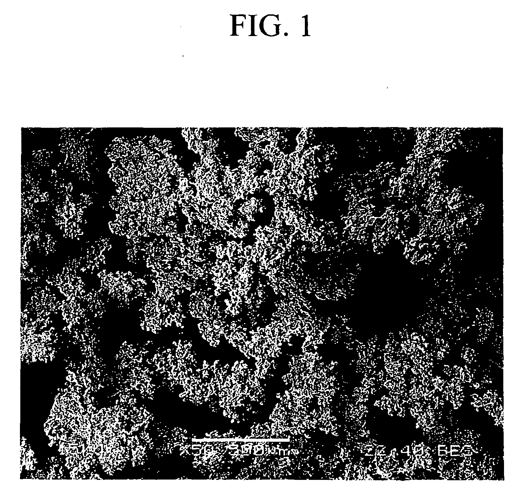

Fig. 1 is an electron micrograph of absorbent resin particles obtained in Example 1.

DETAILED DESCRIPTION OF THE PREFERRED INVENTION

The present invention will now be described in detail.

The absorbent resin used in the present invention contains a polyacidic amino acid as a constituent component of the resin.

Examples of the polyacidic amino acid include polyaspartic acid and polyglutamic acid. These compounds may have a straight-chain or branched structure.

Furthermore, the polyacidic amino acid may have an amide bond, and an amino acid unit other than glutamic acid and aspartic acid in a basic skeleton thereof.

Examples of the amino acid unit other than glutamic acid and aspartic acid include units of aliphatic α-amino acids such as glycine, alanine, valine, leucine, isoleucine, serine, threonine, asparagine, glutamine, lysine, ornithine, cysteine, cystine, methionine, proline, hydroxyproline and arginine; aromatic α-amino acids such as tyrosine, phenylalanine, tryptophan and histidine; amino acids in which a functional group in the side chain of these α-amino acids is substituted; aminocarboxylic acids such as β-alanine and γ-aminobutyric acid; dipeptides (dimers) such as glycyl-glycine and aspartyl-phenylalanine; and tripeptides (trimers) such as glutathione. These amino acids may include optically active substances (L-modification, D- modification) or racemic modification. These amino acid units may exist in the form of a random copolymer or a block copolymer after being combined with glutamic acid or aspartic acid.

The polyacidic amino acid used in the present invention includes a salt thereof. Examples of the salt of the polyacidic amino acid include alkali metal salt, alkali earth metal salt and ammonium salt of the polyacidic amino acid. Examples of the alkali metal salt include sodium salt, potassium salt, lithium salt and rubidium salt, and examples of the alkali earth metal salt include calcium salt and magnesium salt.

The absorbent resin used in the present invention has a structure such that primary particles are agglomerated, and also includes pores having a total pore volume as measured by a mercury penetration method (mercury intrusion porosimetry method) of 0.5 to 5.0 $cm^3/g$, and preferably 0.5 to 3.0 $cm^3/g$.

Inclusion of pores having the total pore volume of 0.5 to 5.0 $cm^3/g$ makes it possible to enhance the wettability to blood and to maintain a structural strength. The total pore surface area of 0.1 $m^2/g$ or more makes it possible to further improve the wettability to blood and to further enhance a blood absorption amount.

In the method described hereinafter, absorbent resin particles are formed by forming primary particles having an average particle diameter of 1 to 50 μm and gradually fusing these primary particles during the manufacturing process. As shown in an electron micrograph of Fig. 1, in the case in which the absorbent resin has a structure such that primary particles are agglomerated, pores are formed in the absorbent resin particles and the surface area of the absorbent resin particles increases, and therefore the wettability to blood can be enhanced.

The pores of the absorbent resin used in the present invention are cavities between primary particles formed by mutual fusion of the primary particles and contribute to adsorption of blood cells and protein in blood and to transfer of blood into absorbent resin particles. These pores generally have an average particle diameter larger than that of the primary particles, and also have diameters of 1 to 100 μm.

The mercury penetration method in the present invention is a method of charging a sample in a container which can be evacuated, evacuating the container to remove moisture and gas, pouring mercury into the container, applying pressure to the mercury, thereby to penetrate the mercury into pores of the sample, and determining the relationship between the pressure applied and the volume of mercury penetrated, thereby determining the size and the volume of the pores.

Specifically, it is a measuring method using a pore diameter measuring device (porosimeter) utilizing a mercury penetration method in the Examples described hereinafter. The pressure applied to mercury is inversely proportional to the diameter of pores into which mercury can be penetrated under pressure and, as the pressure to be applied to mercury increases, mercury penetrates into the smaller pores after penetrating into the larger pores. Therefore, the sizes and the volumes of the pores on the solid surface can be determined by detecting the amount of mercury to be penetrated into the pores while continuously increasing the pressure.

The pores, as used in the mercury penetration method, mean small pores communicating with the outside.

The total pore volume in the present invention is based on a value which is calculated from a value obtained by dividing an integrated value where mercury was penetrated up to a maximum pressure upon measurement by the weight of the material. Also, the total pore surface area in the present invention is based on the value calculated from a relationship between the pressure within the range of the measurement and the amount of mercury penetrated, assuming that the pores have a geometrically cylindrical shape.

To enhance the wettability of blood and to suppress formation of blood clots, the absorbent resin used in the present invention is preferably in the form of particles having an average particle diameter of 100 to 1000 μm.

The average particle diameter in the present invention is based on the numerical value obtained according to the method of measuring the average particle diameter in the Examples described hereinafter.

To enhance the wettability of blood, the absorbent resin used in the present invention preferably has a bulk density of 0.1 to 0.6 g/ml.

The bulk density refers to an apparent density of the material including bubbles and cavities. The numerical value of the bulk density ($\rho_k$) in the present invention is based on the value calculated by the formula (A):

$$\rho_k = \rho\,(1 - \varepsilon)\,(1 - p) \qquad (A)$$

where $\rho$ is the true density of the material, $\varepsilon$ is the void ratio, and p is porosity specific to the material.

The value of the void ratio $\varepsilon$ is a numerical value which can vary depending on the manner of filling a substance into the material.

The absorbent resin used in the present invention preferably contains a vinyl polymer and a polyacidic amino acid as a constituent component of resin particles. The affinity to blood is obtained by the polyacidic amino acid and, furthermore, the blood absorption capacity due to an ion osmotic pressure is imparted by containing the vinyl polymer.

The vinyl polymer is obtained by polymerizing a compound having an ethylenically unsaturated double bond. Examples of the compound having an ethylenically unsaturated double bond include ionic monomers such as (meth) acrylic acid and/or alkali metal salt, alkali earth metal salt and ammonium salt thereof, and 2-(meth)acrylamide-2-methylsulfonic acid and/or alkali metal salt thereof; nonionic monomers such as (meth)acrylamide, N,N-dimethylacrylamide, 2-hydroxyethyl (meth)acrylate and N-methylol(meth)acrylamide; and amino group-containing unsaturated monomers such as diethylaminoethyl (meth)acrylate and dimethylaminopropyl (meth)acrylate, and quaternized compound thereof. Among these compounds, one, two, or more kinds thereof can be used in combination.

Among these compounds, (meth)acrylic acid and/or alkali metal salt, alkali earth metal salt and ammonium salt thereof, and (meth)acrylamide are preferred. Examples of the alkali metal salt include sodium salt, potassium salt, lithium salt and rubidium salt, and examples of the alkali earth metal salt include calcium salt and magnesium salt.

As used herein, the term "(meth)acryl" means "acryl" and "methacryl".

The absorbent resin used in the present invention preferably has such a structure that the polyacidic amino acid having an ethylenically unsaturated double bond is grafted with the vinyl polymer, because the polyacidic amino acid remains in the absorbent resin without being dissociated from the absorbent resin as a result of blood absorption once, and also the absorbent resin is superior in repeated blood absorption properties.

The polyacidic amino acid having an ethylenically unsaturated double bond used in the present invention is not specifically limited and examples thereof include hydrolyzate (i) of polysuccinimide having a maleimide terminal group as a terminal group, and compound (ii) obtained by reacting a polyacidic amino acid with a compound which has an ethylenically unsaturated double bond and a functional group having reactivity with the polyacidic amino acid in a molecule.

The polysuccinimide having a maleimide terminal group can be obtained through maleimide or maleamidic acid after reacting maleic anhydride, fumaric acid or malic acid with ammonia while heating.

The hydrolyzate (i) of polysuccinimide having a maleimide terminal group can be usually obtained by hydrolyzing polysuccinimide obtained above with an aqueous alkali solution added. The reaction temperature is preferably within a range from 0 to 100°C, and more preferably from 20 to 95°C.

Examples of the alkali compound used in the aqueous alkali solution include alkali metal compound and/or alkali earth metal compound. Typical examples of the alkali metal compound or alkali earth metal compound include hydroxide and carbonate, and specific examples thereof include LiOH, NaOH, KOH, $Mg(OH)_2$, $Ca(OH)_2$, $Li_2CO_3$, $Na_2CO_3$, $K_2CO_3$, $MgCO_3$ and $CaCO_3$. Generally, sodium hydroxide or potassium hydroxide is used and an aqueous 0.1-40 wt% solution of these compounds is preferably used. The alkali compound is preferably used in an amount of 0.4 to 1.0 mol per one imide ring group.

The hydrolyzate (i) of polysuccinimide having a maleimide terminal group may be neutralized with a proton acid such as hydrochloric acid, sulfuric acid or phosphoric acid for the purpose of adjusting the pH.

As the polyacidic amino acid used in the compound (ii) obtained by reacting a polyacidic amino acid with a compound which has an ethylenically unsaturated double bond and a functional group having reactivity with the polyacidic amino acid in a molecule, the above polyacidic amino acid can be used.

The compound which has an ethylenically unsaturated double bond and a functional group having reactivity with the polyacidic amino acid in a molecule is not specifically limited, but is preferably a compound represented by the following general formula (2):

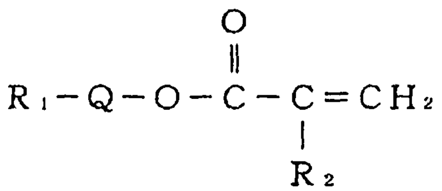

(2)

in the general formula (2), $R_1$ represents at least one kind of a group selected from the group consisting of amino group, epoxy group, carboxyl group, carbodiimide group, oxazoline group, imino group and isocyanate group, Q represents an alkylene group having 1 to 10 carbon atoms, and $R_2$ represents a hydrogen atom or an alkyl group having 1 to 4 carbon atoms, in order to achieve the object of the present invention.

Specific examples of the compound represented by the genearl formula (2) include glycidyl acrylate, glycidyl methacrylate, acrylic acid, methacrylic acid, 2-methacryloxyethyl isocyanate and 2-isocyanate methyl acrylate.

The method of preparing the absorbent resin in the present invention will now be described.

Examples of the method include (a) a method of effecting the water-in-oil type reverse phase suspension polymerization of a polyacidic amino acid (A-1) having at least one ethylenically unsaturated double bond in a molecule and a compound (B) having an ethylenically unsaturated double bond in the presence of a phosphoric ester type surfactant represented by the following general formula (1), and (b) a method of effecting the water-in-oil type reverse phase suspension polymerization of the ethylenically unsaturated compound (B) in the presence of a polyacidic amino acid (A-2) having no ethylenically unsaturated double bond in a molecule in the presence of a phosphoric ester type surfactant represented by the following general formula (1).

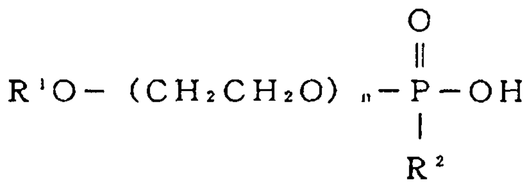

(1)

wherein $R^1$ represents an alkyl group or an alkylaryl group having 8 to 30 carbon atoms, n represents an integer of 1 to 30, and $R^2$ represents a hydroxyl group or $R^1O\text{-}(CH_2CH_2O)_n\text{-}$ ($R^1$ and n are as defined above).

Either of these methods may be used and, when using the above method (a), repeating blood absorption properties of the resulting absorbent resin can be enhanced, and therefore it is preferred.

In the case in which the reaction is effected in the presence of the above phosphoric ester type surfactant, primary particles are agglomerated to form pores, and thus the surface area of resin particles increases and the wettability of blood can be enhanced.

As the polyacidic amino acid having an ethylenically unsaturated double bond(A-1) in the present invention, the above polyacidic amino acid having an ethylenically unsaturated double bond can be used.

Examples of the polyacidic amino acid (A-2) having no ethylenically unsaturated double bond used in the present invention include polyacidic amino acids such as polyaspartic acid and polyglutamic acid.

The phosphoric ester type surfactant used in the present invention has a structure represented by the general formula (1).

In the general formula, $R^1$ represents an alkyl group or an alkylaryl group having 8 to 30 carbon atoms, but is preferably an alkyl group or a monoalkylphenyl group having 8 to 23 carbon atoms in view of industrial availability. Preferred examples of $R^1$ include nonylphenyl group, octylphenyl group, tridecyl group, lauryl group, 2-ethylhexyl group, octadecyl group and dodecylphenyl group.

In the formula, n represents an integer of 1 to 30, but is preferably from 2 to 15. $R^2$ represents a hydroxyl group

or $R^1O-(CH_2CH_2O)_n-$ ($R^1$ and n are as defined above). In the case in which $R^2$ is $R^1O-(CH_2CH_2O)_n-$, two $R^1O-(CH_2CH_2O)_n-$ in a molecule are preferably the same.

A commercially available product of this phosphoric ester type surfactant is commonly a mixture of a phosphate monoester and a phosphate diester.

As specific examples of the compound having an ethylenically unsaturated double bond (B) of the present invention, the above compound having an ethylenically unsaturated double bond can be used.

The compound having an ethylenically unsaturated double bond (B) can exhibit water absorption characteristics by using, as a crosslinking agent, a polyfunctional ethylenically unsaturated compound having two or more ethylenically unsaturated double bonds or a compound having two or more reactive groups in combination.

As the polyfunctional ethylenically unsaturated compound, any compound can be used as long as it is an ethylenically unsaturated compound having two or more ethylenically unsaturated double bonds.

Specific examples thereof include N,N'-methylenebis(meth)acrylamide, (poly)ethylene glycol di(meth)acrylate, (poly)propylene glycol di(meth)acrylate, trimethylolpropane tri(meth)acrylate, trimethylolpropane di(meth)acrylate, glycerin tri(meth)acrylate, glycerin (meth)acrylate, ethylene oxide-modified trimethylolpropane tri(meth)acrylate, pentaerythritol tetra(meth)acrylate and dipentaerythritol hexa(meth)acrylate.

Examples of the compound having two or more reactive groups include polyhydric alcohols such as ethylene glycol, diethylene glycol, triethylene glycol, polyethylene glycol, glycerin, polyglycerin, propylene glycol, 1,4-butanediol, 1,5-pentanediol, 1,6-hexanediol, neopentyl alcohol, diethanolamine, tridiethanolamine, polypropylene glycol, polyvinyl alcohol and pentaerythritol, sugar alcohols such as sorbitol and sorbitan, and saccharides such as glucose, mannitol, mannitan, sucrose and glucose; polyglycidyl ethers such as ethylene glycol diglycidyl ether, polyethylene glycol diglycidyl ether and glycerin triglycidyl ether; haloepoxy compounds such as epichlorohydrin and $\alpha$-methylchlorohydrin; polyaldehydes such as glutaraldehyde and glyoxal; polyamines such as ethylenediamine; and hydroxides, halides, carbonates, oxides and borates such as borax of metals of the groups 2A, 3B and 8 of the Periodic Table, such as calcium hydroxide, calcium chloride, calcium carbonate, calcium oxide, magnesium borax chloride, magnesium oxide, aluminum chloride, zinc chloride and nickel chloride, and polyvalent metal compounds such as aluminum isopropylate.

One, two, or more kinds of these polyfunctional ethylenically unsaturated compounds having two or more ethylenically unsaturated groups, or these compounds having two or more reactive groups can be used taking into account the reactivity.

Furthermore, the method of preparing an absorbent resin used in the present invention will now be described in detail.

The absorbent resin used in the present invention can be prepared by subjecting polymer particles, which are obtained by supplying an aqueous solution containing the above phosphoric ester type surfactant, a polyacidic amino acid having an ethylenically unsaturated double bond (A-1) in a molecule and/or a polyacidic amino acid (A-2) containing no ethylenically unsaturated double bond in a molecule, a compound having an ethylenically unsaturated double bond (B) and a crosslinking agent (hereinafter referred to as an aqueous solution of an ethylenically unsaturated compound (B)) and a radical initiator into an inert solvent containing a phosphoric ester type surfactant represented by the general formula (1) and effecting the water-in-oil type reverse phase suspension polymerization, to a surface crosslinking treatment.

The inert solvent used in the present invention means a hydrophobic solvent which is slightly soluble in water. Such an inert solvent may be any one as long as it is inert in the polymerization reaction in the case of preparing resin particles in the present invention, and it is not specifically limited. Examples of the inert solvent include aliphatic hydrocarbons such as n-pentane, n-hexane, n-heptane and n-octane; alicyclic hydrocarbons such as cyclohexane and methylcyclohexane; and aromatic hydrocarbons such as benzene, toluene and xylene. Among these solvents, aliphatic hydrocarbons such as n-hexane, n-heptane and cyclohexane or alicyclic hydrocarbons are preferred because an absorbent resin free of tackiness can be obtained.

The amount of the phosphoric ester type surfactant in the inert solvent is preferably within a range from 0.01 to 5% by weight. When the amount is within the above range, a desired dispersion effect can be obtained without lowering blood absorption characteristics of the present invention.

The amount of the inert solvent is preferably 0.5-10 times by weight larger than that of the aqueous solution of the ethylenically unsaturated compound (B) used in the reaction.

The phosphoric ester type surfactant is preferably added to the aqueous ethylenically unsaturated compound (B) solution so that a ratio of a concentration (X) of the phosphoric ester type surfactant in the solvent to a concentration (Y) of the phosphoric ester type surfactant in the aqueous solution of the ethylenically unsaturated compound (B), (X/Y), satisfies the following expression:

$$0 < X/Y \leqq 10$$

When the amount of the phosphoric ester type surfactant in the aqueous solution of the ethylenically unsaturated compound (B) is within the above range, the average particle diameter of the resulting absorbent resin can be controlled to 100 to 1000 μm, and thus blood absorption characteristics can be improved.

Regarding the surfactant in the aqueous ethylenically unsaturated compound (B) solution, the phosphoric ester type surfactant can be used in combination with an anionic surfactant and/or a nonionic surfactant for the purpose of optionally controlling the surface structure of the resulting absorbent resin more complicatedly.

Examples of the anionic surfactant include anionic surfactants such as sodium polyoxyethylene lauryl ether sulfate, sodium polyoxyethylene alkyl ether sulfate, sodium polyoxyethylene alkyl phenyl sulfate, sodium lauryl sulfate, trietanolamine lauryl sulfate, ammonium lauryl sulfate, sodium dodecylbenzenesulfonate and sodium dialkyl sulfosuccinate. Examples of the nonionic surfactant include nonionic surfactants such as polyoxyethylene lauryl ether, polyoxyethylene cetyl ether, polyoxyethylene stearyl ether, polyoxyethylene oleyl ether and poly(ethylene glycol fatty acid ester).

For the purpose of controlling the surface structure of absorbent resin particles, more complicatedly, a small amount of water-soluble polymers such as hydroxyethylcellulose, polyacrylic acid and polyvinyl alcohol may be added in the aqueous ethylenically unsaturated compound (B) solution.

Examples of the radical polymerization initiator include inorganic peroxides, such as for example, hydrogen peroxide, ammonium persulfate, potassium persulfate and sodium persulfate, organic peroxides, such as for example, benzoyl peroxide, di-t-butyl peroxide, cumene hydroxyperoxide, succinic acid peroxide and di(2-ethoxyethyl)peroxydicarbonate, azo compounds, such as for example, azobisisobutylonitrile, azobiscyanovaleric acid and 2,2'-azobis(2-aminopropane)hydrochloride and redox catalysts, such as for example, combinations of reducing agents such as sulfite or bisulfite of alkali metal, ammonium sulfite, ammonium bisulfite and ascorbic acid, and oxidizing agents such as persulfate of alkali metal, ammonium persulfate and peroxide).

The phosphoric ester type surfactant in the inert solvent and the phosphoric ester type surfactant in the aqueous solution of the ethylenically unsaturated compound (B) may be the same or different.

The water-in-oil type reverse phase suspension polymerization used in the present invention is effected by supplying the aqueous solution of the ethylenically unsaturated compound (B) containing the phosphoric ester type surfactant into the inert solvent containing the phosphoric ester type surfactant, thereby to disperse the aqueous solution in the form of droplets into oil and to polymerize the suspension.

The polymerization reaction may be initiated after supplying the entire aqueous solution of the ethylenically unsaturated compound (B) into the inert solvent, or the aqueous solution of the ethylenically unsaturated compound (B) may be gradually supplied in several portions during the polymerization, but the latter method of gradually supplying in several portions is preferred. According to the former method of initiating the polymerization reaction after supplying the entire aqueous solution of the ethylenically unsaturated compound (B) into the inert solvent, the range capable of producing desired absorbent resin particles is narrowed and it becomes difficult to eliminate heat generation caused by the polymerization.

In contrast, according to the latter method, the polymerization is initiated after supplying a portion, i.e., 1 to 25% of the aqueous solution of the ethylenically unsaturated compound (B) into the inert solvent and, after the polymerization of the compound proceeded to some extent, the polymerization is effected while gradually supplying the remaining aqueous solution of the ethylenically unsaturated compound (B).

As another method other than the methods described above, the polymerization may be allowed to proceed simultaneously while gradually supplying the aqueous solution of the ethylenically unsaturated compound (B) into the inert solvent set previously under the polymerization conditions from the beginning.

In the case of effecting these methods, a mixture of the aqueous solution of the ethylenically unsaturated compound (B) and a portion of the inert solvent may be used as the aqueous solution of the ethylenically unsaturated compound (B) and the mixture may be supplied into the remaining inert solvent.

The aqueous solution of the ethylenically unsaturated compound (B) is commonly supplied for 20% or more, and preferably 40% or more of the entire polymerization time.

Although the aqueous solution of the ethylenically unsaturated compound (B) is commonly supplied at a constant rate, if necessary, it may vary and supply can be temporarily interrupted during the polymerization. For example, the polymerization is initiated by continuously supplying the aqueous solution of the ethylenically unsaturated compound (B) into a hydrophobic organic solvent under the polymerization conditions and, when 1 to 25% of the aqueous solution of the ethylenically unsaturated compound (B) was supplied, only the polymerization is allowed to proceed by stopping supply of the aqueous solution of the ethylenically unsaturated compound (B) for 3 to 60 minutes, preferably 5 to 30 minutes, and then the aqueous solution of the ethylenically unsaturated compound (B) can be supplied again at the same rate as described above. This method is one of preferred embodiments to produce the absorbent resin of the present invention.

The polymerization temperature varies depending on the polymerization initiator, but is usually within a range from 40 to 150°C. In the case in which the polymerization temperature is too high, self-crosslinking proceeds and the water absorption capability of the resulting resin particles is lowered. On the other hand, when the polymerization

temperature is too low, not only does the polymerization require a long time, but also unexpected polymerization is likely to occur to form an agglomerate. Preferred polymerization temperature is within a range from 60 to 90°C and the polymerization is preferably effected under the reflux conditions of the inert solvent.

Examples of the method of adding the polyacidic amino acid having at least one ethylenically unsaturated double bond (A-1) in a molecule or the polyacidic amino acid (A-2) having no ethylenically unsaturated double bond in a molecule into the inert solvent include, but are not limited to, (1) a method of previously mixing the aqueous solution of the polyacidic amino acid having at least one ethylenically unsaturated double bond (A-1) in a molecule or the polyacidic amino acid (A-2) having no ethylenically unsaturated double bond in a molecule with the aqueous solution of the ethylenically unsaturated compound (B) and adding the mixture; (2) a method of simultaneously adding with the aqueous solution of the ethylenically unsaturated compound (B); and (3) a method of adding after adding the aqueous solution of the ethylenically unsaturated compound (B).

Although any method may be used, the method (3) is preferably used because the stability of the system can be maintained.

In the case in which the polyacidic amino acid having at least one ethylenically unsaturated double bond (A-1) in a molecule or the polyacidic amino acid (A-2) having no ethylenically unsaturated double bond in a molecule is added after adding the aqueous solution of the ethylenically unsaturated compound (B), the aqueous solution thereof is added as it is, or is added after adding an inert solvent dissolved with a surfactant, to the aqueous solution of the polyacidic amino acid having at least one ethylenically unsaturated double bond (A-1) in a molecule or the polyacidic amino acid (A-2) having no ethylenically unsaturated double bond in a molecule, and dispersing them with stirring. The latter method is preferred because the resin particles are not mutually agglomerated and the polymerization stability is improved.

The surfactant to be dissolved in the aqueous solution of the polyacidic amino acid having at least one ethylenically unsaturated double bond (A-1) in a molecule or the polyacidic amino acid (A-2) having no ethylenically unsaturated double bond in a molecule is not specifically limited and one, two, or more kinds of phosphoric ester type surfactants used in the reverse phase suspension polymerization method can be used.

The absolute value of the stirring rate among the stirring conditions in the reverse phase suspension polymerization varies depending on the kind of mixing impeller used and the size of a polymerization reaction tank, and therefore it cannot be shown unambiguously. Since the stirring speed exerts an influence on the average particle diameter of the resin particles and the average particle diameter is preferably from 100 to 1000 μm to achieve the object of the present invention, the stirring rate is preferably within a range from 100 to 1000 rpm, and more preferably from 200 to 1000 rpm. By appropriately selecting the kind of the mixing impeller and the mixing power within the above range, it becomes possible to obtain absorbent resin particles which have such a structure that primary particles are agglomerated, and also has a large area wetted with blood.

By the above reverse phase suspension polymerization method, a mixture of slurry-like absorbent resin particles comprising a hydrous gel, an excess surfactant and an inert solvent can be produced. The gel-like absorbent resin particles can be obtained from this slurry-like mixture through a known method, for example, direct dehydration or azeotropic dehydration with an inert solvent, drying and classification with a sieve.

The absorbent material of the present invention is preferably subjected to the crosslinking reaction in the vicinity of the resulting absorbent resin particles using a surface crosslinking agent. The osmotic pressure to blood can be further enhanced by crosslinking the vicinity of the surface of the resin particles, and thus it is made possible to further enhance absorption characteristics to blood.

Examples of the surface crosslinking agent include compounds two or more functional groups capable of reacting with a functional group in the vicinity of the absorbent resin particles. Since the surface crosslinking agent remains on the surface of the particles when used in the article for blood absorption, compounds which are very safe for the human body are preferred.

Examples of the compound include compounds having a reactive group capable of reacting with two or more carboxyl group (carboxylate groups), such as polyamine and polyglycidyl ether; silane coupling agents such as γ-glycidoxypropyltrimethoxysilane, γ-glycidoxypropylmethyldiethoxysilane, N-β-(aminoethyl)-γ-aminopropyltrimethoxysilane and γ-mercaptopropyltrimethoxysilane; silanol condensing catalysts such as dibutyltin dilaurate, dibutyltin diacetate and dibutyltin dioctoate; and ethylenically unsaturated compounds having a reactive group, such as glycidyl methacrylate. One, two, or more kinds thereof can be used in combination.

The surface crosslinking of the absorbent resin particles can be effected by mixing the powdered resin particles, which was made from the slurry-like mixture by azeotropic dehydration, or directly dehydrating using a suitable method such as heating until a predetermined water content is attained, with a surface crosslinking agent. At this time, water and a hydrophilic solvent are preferably used to uniformly mix the resin particles with the surface crosslinking agent. 50 Parts by weight or less of water and 60 parts by weight or less of the hydrophilic solvent are mixed and used based on 100 parts by weight of the resin.

Examples of the hydrophilic solvent include lower alcohols such as methanol, ethanol, n-propanol, isopropanol,

n-butanol and isobutanol; ketones such as acetone and methyl ethyl ketone; ethers such as dioxane, tetrahydrofuran and diethyl ether; amides such as N,N-dimethylformamide and N,N-diethylformamide; and sulfoxides such as dimethyl sulfoxide.

The method of mixing the particles with the surface crosslinking agent is not specifically limited and, for example, a known mixing device can be used.

Examples of the known mixing device include mixing devices such as cylindrical mixer, double wall conical mixer, high-speed mixer, twin-cylinder mixer, ribbon mixer, screw mixer, fluid type furnace rotary disk mixer, air current mixer, double-arm kneader, internal mixer, grinding kneader, rotary mixer and screw extruder. In the case of mixing using these mixing devices, the surface crosslinking agent is preferably added while stirring the resin particles and, more preferably, mixing is effected while spraying the surface crosslinking agent.

In the case of surface crosslinking, the heating time is appropriately selected according to the heating temperature and is preferably within a range from 5 minutes to 100 hours at a temperature of 60 to 300°C so as to obtain absorbent resin particles having high water absorption properties without causing thermal deterioration.

The heating device used in the case of heating is not specifically limited, but a dryer or heating oven can be commonly used. Specific examples thereof include channel mixing dryer, rotary dryer, disk dryer, fluidized layer dryer, air current dryer, infrared dryer and vacuum dryer.

The absorbent material of the present invention exhibits excellent absorption characteristics to blood. The blood absorption is not specifically limited, but is preferably 6 g/g or more.

The absorbent article of the present invention will now be described.

The absorbent article of the present invention comprises an absorbent core including an absorbent material and a fiber material, and a sheet-like material provided on both surfaces of the absorbent core, wherein absorbent material is an absorbent material comprising, as a main component, an absorbent resin which contains a polyacidic amino acid as a constituent component of the resin and has such a structure that primary particles are agglomerated, and also includes pores having a total pore volume as measured by a mercury penetration method of 0.5 to 5.0 $cm^3/g$.

Examples of the sheet-like material constituting the absorbent article of the present invention include nonwoven fabric, woven fabric, synthetic films made of materials such as polyethylene, polypropylene, ethylene-vinyl acetate, polyvinyl chloride and polyamide; films made of composite materials comprising the synthetic resin and a nonwoven or woven fabric; and fiber material sheets described hereinafter.

As the absorbent material constituting the absorbent article of the present invention, the absorbent materials described above can be used.

The fiber material constituting the absorbent article of the present invention includes, for example, hydrophobic fiber material or hydrophilic fiber material, but the hydrophilic fiber material is preferred because of excellent affinity to blood. Examples of the hydrophilic fiber material include cellulose fibers such as mechanical pulp obtained from lumber, and semi-chemical pulp; artificial cellulose fibers such as rayon and cellulose acetate; and fiber materials obtained by hydrophilization of thermoplastic resin.

The shape of the fiber material is not specifically limited, but fibers, and sheets such as tissue paper and pulp mats, can be optionally selected.

Specific examples of the method of producing the absorbent article include a method of interposing the absorbent core between two sheet-like materials, and bonding the outer peripheral portion of the sheet-like material using an adhesive such as hot melt type adhesive, or a bonding means such as heat seal.

Examples of the method of producing an absorbent core comprising an absorbent material and a fiber material include (1) a method of piling up fiber materials in the form of a sheet to obtain a fiber sheet and folding the resulting fiber sheet, thereby to wrap an absorbent material, (2) a method of piling up fiber materials in the form of a sheet to obtain a fiber sheet, scattering an absorbent material on the resulting fiber sheet, coating the fiber sheet thereon and integrally laminating them, (3) a method of scattering an absorbent material on a multi-layered fiber sheet, and (4) a method of mixing a fiber material with an absorbent material and piling up the resulting mixture in the form of a sheet.

Examples of the absorbent article include articles requiring blood absorption characteristics, such as sanitary napkins, tampons, medical blood absorption sheets, drip in the field of fresh food or sea food, wound protectors, wound healing materials, and surgical waste liquid treating agents. The absorbent articles exhibits excellent absorption characteristics to water containing proteins, for example, cow's milk, human milk and vaginal discharges, similar to blood, and also exhibits excellent absorption characteristics to urine, seawater, cement waste water, soil water, fertilizer-containing water, rainwater and drainage, like a conventional absorbent material. Therefore, the absorbent article can be widely applied in various fields.

## Examples

The present invention will now be described in more detail by way of Examples and Comparative Examples. In the following Examples and Comparative Examples, percentages are by weight unless otherwise specified. Various

properties of the materials were determined by the procedures described generally below. The charge compositions of Examples 1 to 3, Comparative Example 1 and Comparative Example 2 are summarized in Table 1.

(Procedure for the measurement of blood absorption amount)

On 15 pieces of toilet paper (55 mm × 75 mm) which are laid one upon another and impregnated with 20 ml of HORSE WHOLE BLOOD DEFIBRINATED (available from NIPPON BIO-SUPP. CENTER) in a petri dish having an inner diameter of 95 mm, about 1 g of absorbent resin particles obtained in the Examples described hereinafter were placed and, after allowing it to absorb liquid for 5 minutes, the swollen gel of the resin was collected and weighed. The weight of the swollen gel after liquid absorption was divided by the weight of resin particles before liquid absorption to calculate a blood absorption amount (g/g).

(Procedure for the measurement of total pore volume and total pore surface area)

Using the absorbent resin particles obtained in the Examples described hereinafter as samples, the measurement was effected by Poresizer 9320 (Micromeretics porosimeter, pore diameter measuring device by a mercury penetration method, manufactured by Shimadzu Corporation).

(Procedure for the measurement of average particle diameter)

About 20 g of each of absorbent resin particles obtained in the Examples described hereinafter was put in an uppermost sieve among a sieve having a pore diameter of 16 mesh (1000 μm), a sieve having a pore diameter of 30 mesh (500 μm), a sieve having a pore diameter of 100 mesh (150 μm), a sieve having a pore diameter of 140 mesh (106 μm), a sieve having a pore diameter of 235 mesh (63 μm) (JIS-Z8801) and a pan, which are combined in this sequence, followed by sufficient shaking. After the weight of the resin particles remaining in each sieve was measured, a particle size distribution was determined from a weight fraction based on 100% of the total weight and a 50% particle diameter on the weight basis was taken as an average particle diameter.

(Procedure for the measurement of bulk density)

Using the absorbent resin particles obtained in the Examples described hereinafter, the measurement was effected according to JIS·K-6721. The measurement was effected three times and an average of the resulting values was taken as a value of the bulk density.

(Procedure for the measurement of amount of blood returned)

On the absorbent sheets obtained in the Examples described hereinafter, about 3 g of HORSE WHOLE BLOOD DEFIBRINATED was dropped from the upper portion using a dropping pipet and, after a lapse of about 2 minutes, 4 pieces of filter paper, which are laid one upon another, were laid on the surface and loading was effected by placing 1000 g of a weight thereon. After 10 seconds, the amount of blood transferred to filter paper was measured and the resulting value was taken as an amount of blood returned. Reference Example 1 (Preparation Example of polysuccinimide)

In a 1 L four-necked flask equipped with a stirrer, a thermometer, a reflux condenser and a nitrogen gas introducing device, 96 g of maleic anhydride and 50 g of deionized water were added. Maleic anhydride was dissolved by heating to 55°C, followed by cooling to obtain a slurry of maleic anhydride. The system was heated again and, after the temperature reached 55°C, 60.8 g of 28% aqueous ammonia was added. Then, the temperature in the system was heated to 80°C. After the reaction was effected for 3 hours, the resulting aqueous solution was dried to obtain a reaction intermediate. In a 2 L Kjeldal flask, 100 g of the reaction intermediate and 10 g of 85% phosphoric acid were charged and the reaction was effected in an oil bath at 200°C under reduced pressure for 4 hours using an evaporator. The resulting product was washed with water and ,methanol several times. The weight-average molecular weight of the resulting polysuccinimide was measured by GPC. As a result, it was 3000.

Example 1

In a 500 ml four-necked flask equipped with a stirrer, a thermometer, a reflux condenser and a nitrogen gas introducing device, 75 g of an aqueous solution prepared by dissolving 20.6 g of sodium hydroxide was charged and 50 g of powder of polysuccinimide obtained in Reference Example 1 was added to obtain an aqueous solution of polysuccinimide. After raising the temperature to 90°C, 5.0 g of glycidyl methacrylate was added and the reaction was

effected for one hour to obtain an aqueous solution containing a hydrolyzate of polysuccinimide having a methacryloyl group introduced therein.

In a 500 ml Erlenmeyer flask, 30 g of acrylic acid was charged and 81.5 g of an aqueous lithium hydroxide solution prepared by dissolving 8.74 g of lithium hydroxide monohydrate was added dropwise while cooling from the outside, thereby to neutralize 50 mol% of acrylic acid. In the solution, 1.12 g of PLYSURF™ A210G (phosphoric ester type surfactant polyoxyethylene octylphenyl ether phosphoric ester, manufactured by DAI-ICHI KOGYO SEIYAKU CO., LTD.) was added and dissolved. Furthermore, 23.4 mg of N,N'-methylenebis acrylamide and 0.05 g of ammonium persulfate were added and dissolved in this solution.

Separately, in a 500 ml four-necked flask equipped with a stirrer, a thermometer, a reflux condenser and a nitrogen gas introducing device, 164 g of cyclohexane was charged and 0.82 g of PLYSURF™ A210G was added and dispersed while stirring at 500 rpm. After replacing the atmosphere in the flask with nitrogen, the temperature was raised to 75°C and the aqueous acrylic acid solution prepared above was added dropwise over 60 minutes. After the completion of the dropwise addition, 7.8 g of the aqueous solution containing a hydrolyzate of polysuccinimide having a methacryloyl group introduced therein was added at a time. After maintaining at 70 to 75°C for 3 hours, dehydration was effected until the water content of the resin produced by azeotropy with cyclohexane reached 10%. Stirring was effected at a constant revolution number of 500 rpm. After the completion of the reaction, the cyclohexane phase was separated by decantation and water was removed from the resulting hydrous resin particles by vacuum drying to obtain a polymer powder.

After weighing 30 g of the resulting polymer particles in a 500 ml flask, a mixed solution of 1.2 g of acetone, 2.1 g of deionized water, 0.09 g of glycidyl methacrylate and 0.09 g of ammonium persulfate and 0.3 g of hydrophilic silica (manufactured by Nippon Aerosil Co., Ltd., 200CF) were uniformly scattered. The hydrous resin particles thus obtained were vacuum-dried at 108°C for one hour, thereby to effect surface crosslinking of the resin particles. The resulting absorbent resin particles were observed by an electron microscope. As a result, the absorbent resin particles had a structure such that primary particles were agglomerated as shown in Fig. 1. The total pore volume, the total pore surface area, the average particle diameter and the bulk density of the resulting resin particles were as shown in Table 1.

The evaluation results of characteristics of the absorbent materials obtained from the above absorbent resin particles of the present invention are shown in Table 1. As is apparent from Table 1, the absorbent material obtained in Example 1 is superior in blood absorption capacity and has good affinity to blood.

Example 2

Absorbent resin particles were obtained by the same operation as in Example 1 was repeated, except that the amount of N,N'-methylenebisacrylamide was changed to 93.6 mg. The resulting absorbent resin particles were observed by an electron microscope. As a result, the absorbent resin particles had such a structure that primary particles are agglomerated. The total pore volume, the total pore surface area, the average particle diameter and the bulk density of the resulting resin particles are as shown in Table 1. The evaluation results of characteristics of the absorbent materials obtained from the above absorbent resin particles of the present invention are shown in Table 1. As is apparent from Table 1, the absorbent material obtained in Example 2 is superior in blood absorption capacity and has good affinity to blood.

Example 3

Absorbent resin particles were obtained by the same operation as in Example 1 was repeated, except that 0.84 g of PLYSURF™ AL (polyoxyethylene distyrenated phenyl ether phosphoric ester, manufactured by DAI-ICHI KOGYO SEIYAKU CO., LTD.) was added to an aqueous lithium hydroxide-neutralized solution of acrylic acid in place of PLYSURF™ A210G and 0.41 g of PLYSURF™ A212C (polyoxyethylene tridecyl ether phosphoric ester, manufactured by DAI-ICHI KOGYO SEIYAKU CO., LTD.) was added to cyclohexane in place of PLYSURF™ A21 0G. The resulting absorbent resin particles were observed by an electron microscope. As a result, the absorbent resin particles had a structure such that primary particles were agglomerated. The total pore volume, the total pore surface area, the average particle diameter and the bulk density of the resulting resin particles are as shown in Table 1. The evaluation results of characteristics of the absorbent materials obtained from the above absorbent resin particles of the present invention are shown in Table 1. As is apparent from Table 1, the absorbent material obtained in Example 3 is superior in blood absorption capacity and has good affinity to blood.

Example 4

Absorbent resin particles were obtained by the same operation as in Example 1 was repeated, except that 1.68 g of PLYSURF™ A213B (polyoxyethylene lauryl ether phosphoric ester, manufactured by DAI-ICHI KOGYO SEIYAKU CO., LTD.) and 0.78 g of Emerl™ 20C (polyoxyethylene lauryl sulfate ester, manufactured by Kao Corporation) were

added to an aqueous lithium hydroxide-neutralized solution of acrylic acid in place of PLYSURF™ A210G and 0.41 g of PLYSURF™ AL was added to cyclohexane in place of PLYSURF™ A210G. The resulting absorbent resin particles were observed by an electron microscope. As a result, the absorbent resin particles had a structure such that primary particles were agglomerated. The total pore volume, the total pore surface area, the average particle diameter and the bulk density of the resulting resin particles are as shown in Table 1. The evaluation results of characteristics of the absorbent materials obtained from the above absorbent resin particles of the present invention are shown in Table 1. As is apparent from Table 1, the absorbent material obtained in Example 4 is superior in blood absorption capacity and has good affinity to blood.

Example 5

In a 500 ml four-necked flask equipped with a stirrer, a thermometer, a reflux condenser and a nitrogen gas introducing device, 75 g of an aqueous solution prepared by dissolving 20.6 g of sodium hydroxide was charged and 50 g of powder of polysuccinimide obtained in Reference Example 1 was added to obtain an aqueous solution containing a hydrolyzate of polysuccinimide. Absorbent resin particles were obtained by the same operation as in Example 1, except that the aqueous solution containing a hydrolyzate of polysuccinimide obtained by this operation was used in place of the aqueous solution containing a hydrolyzate of polysuccinimide having a methacryloyl group introduced therein of Example 1. The resulting absorbent resin particles were observed by an electron microscope. As a result, the absorbent resin particles had a structure such that primary particles were agglomerated. The total pore volume, the total pore surface area, the average particle diameter and the bulk density of the resulting resin particles were as shown in Table 1. The evaluation results of characteristics of the absorbent materials obtained from the above absorbent resin particles of the present invention are shown in Table 1. As is apparent from Table 1, the absorbent material (Example 5) of the present invention obtained from the absorbent resin particles is superior in blood absorption capacity and has good affinity to blood.

Comparative Example 1

In a 500 ml Erlenmeyer flask, 30 g of acrylic acid was charged and 81.5 g of an aqueous lithium hydroxide solution prepared by dissolving 8.74 g of lithium hydroxide monohydrate was added dropwise while cooling from the outside, thereby to neutralize 50 mol% of acrylic acid. In the solution, 23.4 mg of N,N'-methylenebisacrylamide was added and 0.05 g of ammonium persulfate was further added and dissolved.

Separately, in a 500 ml four-necked flask equipped with a stirrer, a thermometer, a reflux condenser and a nitrogen gas introducing device, 164 g of cyclohexane was charged and 0.82 g of PLYSURF™ A210G was added and dispersed while stirring at 500 rpm. After replacing the atmosphere in the flask with nitrogen, the temperature was raised to 75°C and the aqueous acrylic acid solution prepared above was added dropwise over 60 minutes.

After maintaining at 70 to 75°C for 3 hours, dehydration was effected until the water content of the resin produced by azeotropy with cyclohexane reached 10%. Stirring was effected at a constant revolution number of 500 rpm. After the completion of the reaction, the cyclohexane phase was separated by decantation and water was removed from the resulting hydrous resin particles by vacuum drying to obtain a polymer powder.

After weighing 30 g of the resulting polymer particles in a 500 ml flask, a surface crosslinking treatment was effected by the same operation as in Example 1. The resulting absorbent resin particles were observed by an electron microscope. As a result, the absorbent resin particles had such a structure that primary particles were agglomerated; however, excellent blood absorption capacity could not be obtained as shown in Table 1. The total pore volume, the total pore surface area, the average particle diameter and the bulk density of the resulting resin particles are as shown in Table 1.

Comparative Example 2

The total pore volume, the total pore surface area and the bulk density of Aquaric™ CA-K4 (polyacrylic acid crosslinked body, manufactured by NIPPON SHOKUBAI CO., LTD.) are shown in Table 1. The average particle diameter measured by the above procedure for the measurement of the average particle diameter is as shown in Table 1.

Comparative Example 3

10 g of Aquaric™ CA-K4 was swollen by allowing to absorb an aqueous solution prepared by dissolving 0.2 g of poly ethylene glycol (molecular weight: 600) in 150 g of deionized water. The swollen resin was transferred to a Kjeldal flask and then frozen using liquid nitrogen. Then, the swollen resin was freeze-dried for 72 hours by putting the Kjeldal flask containing the frozen swollen resin into a freeze-dryer. The resulting resin was ground to obtain absorbent resin

particles capable of passing through a sieve having a pore diameter of 1000 μm. The total pore volume, the total pore surface area and the bulk density of the absorbent resin particles are shown in Table 1. The absorbent material made of the absorbent resin was placed on toilet paper impregnated with blood. As a result, wetting of the entire resin with blood was not observed, although rapid blood absorption was observed at the portion contacted with the surface of toilet paper. The portion wetted with blood was collected. As a result, the bulky structure was collapsed as a result of wetting with blood.

Table 1

| | Total pore volume ($cm^3/g$) | Total pore surface area ($m^2/g$) | Average particle diameter (μm) | Bulk density (g/ml) | Blood absorption amount (g/g) |
|---|---|---|---|---|---|
| Example 1 | 0.804 | 0.215 | 320 | 0.34 | 11.5 |
| Example 2 | 0.827 | 0.224 | 350 | 0.32 | 12.8 |
| Example 3 | 1.307 | 0.287 | 200 | 0.50 | 11.2 |
| Example 4 | 0.962 | 0.215 | 350 | 0.34 | 11.2 |
| Example 5 | 0.785 | 0.202 | 320 | 0.34 | 10.5 |
| Comparative Example 1 | 0.431 | 0.205 | 330 | 0.33 | 4.2 |
| Comparative Example 2 | 0.425 | 0.073 | 400 | 0.67 | 2.5 |
| Comparative Example 3 | 5.312 | 0.813 | - | 0.12 | 3.5 |

Example 6

On a polyethylene sheet cut to a size of 4 cm × 5 cm, 0.075 g of a pulp sheet (weight: 37.5 $g/m^2$) cut to a size of 4 cm × 5 cm was placed and, furthermore, 0.3 g of the absorbent material (weight: 150 $g/m^2$) obtained in Example 1 was uniformly piled up thereon. An absorbent sheet was assembled by placing 0.075 g of a pulp sheet cut to a size of 4 cm × 5 cm thereon and wrapping with a tape. With respect to the resulting absorbent sheet, the measurement was effected by the procedure for the measurement of amount of blood returned. As a result, the amount of blood returned to filter paper was 0.087 g.

The absorbent material and the absorbent article of the present invention can be used for various purposes requiring blood absorption characteristics, for example, sanitary napkins, tampons, medical blood-absorbent sheets and drip absorbents because of excellent absorption characteristics of blood and absorbency of water containing proteins.

## Claims

1. An absorbent material comprising an absorbent resin containing a polyacidic amino acid as a constituent component of the resin and having a structure in which primary particles are agglomerated **characterized by** including pores having a total pore volume as measured by a mercury penetration method (a mercury intrusion porosimetry method) of 0.5 to 5.0 $cm^3/g$.

2. The absorbent material according to claim 1, wherein the pores of the absorbent resin have a total pore surface area of 0.1 $m^2/g$ or more.

3. The absorbent material according to claim 1 or 2, wherein the absorbent resin contains a vinyl polymer and a polyacidic amino acid as the constituent component of the resin.

4. The absorbent material according to claim 3, wherein the absorbent resin has a structure in which a polyacidic amino acid having an ethylenically unsaturated double bond is grafted with the vinyl polymer.

5. The absorbent material according to claim 1 or 2, wherein the absorbent resin has a bulk density of 0.1 to 0.6 g/ml.

6. The absorbent material according to claim 1 or 2, wherein the absorbent resin is in the form of particles having a average particle diameter of 100 to 1,000 μm.

7. The absorbent material according to claim 1 or 2, wherein the absorbent resin is obtained by effecting the water-in-oil type reverse phase suspension polymerization of a polyacidic amino acid (A-1) having at least one ethylenically unsaturated double bond in a molecule and/or a polyacidic amino acid (A-2) having no ethylenically unsaturated double bond in a molecule and a compound (B) having at least one ethylenically unsaturated double bond in a molecule in the presence of a phosphoric ester type surfactant represented by the following general formula (1):

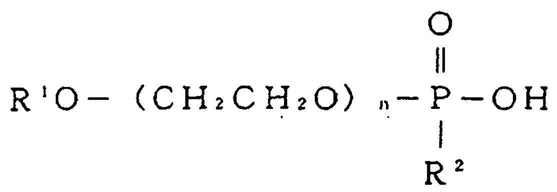 (1)

wherein $R^1$ represents an alkyl group or an alkylaryl group having 8 to 30 carbon atoms, n represents an integer of 1 to 30, and $R^2$ represents a hydroxyl group or $R^1O\text{-}(CH_2CH_2O)_{n}\text{-}$($R^1$ and n are as defined above).

8. An absorbent article comprising an absorbent core including an absorbent material and a fiber material, and a sheet-like material provided on both surfaces of the absorbent core, wherein the absorbent material is an absorbent material according to claim 1.

**Patentansprüche**

1. Absorptionsmaterial umfassend ein Absorptionsharz enthaltend eine mehrbasige Aminosäure als konstituierende Komponente des Harzes und mit einer Struktur, in der primäre Teilchen agglomeriert sind, **gekennzeichnet durch** den Gehalt an Poren mit einem Gesamtporenvolumen von 0,5 bis 5,0 $cm^3$/g gemessen nach einer Quecksilber-Penetrationsmethode (Quecksilber-Intrusionsporosimetrie-Methode).

2. Absorptionsmaterial nach Anspruch 1, worin die Poren des Absorptionsharzes eine Gesamtporenoberfläche von 0,1 $m^2$/g oder mehr aufweisen.

3. Absorptionsmaterial nach Anspruch 1 oder 2, worin das Absorptionsharz ein Vinylpolymer und eine mehrbasige Aminosäure als konstituierende Komponenten des Harzes enthält.

4. Absorptionsmaterial nach Anspruch 3, worin das Absorptionsharz eine Struktur aufweist, in der eine mehrbasige Aminosäure mit einer ethylenisch ungesättigten Doppelbindung mit dem Vinylpolymer gepfropft ist.

5. Absorptionsmaterial nach Anspruch 1 oder 2, worin das Absorptionsharz eine Schüttdichte bzw. Rohdichte von 0,1 bis 0,6 g/ml aufweist.

6. Absorptionsmaterial nach Anspruch 1 oder 2, worin das Absorptionsharz in Form von Teilchen mit einem durchschnittlichen Teilchendurchmesser von 100 bis 1.000 μm vorliegt.

7. Absorptionsmaterial nach Anspruch 1 oder 2, worin das Absorptionsharz erhalten wird, indem eine Umkehrphasen-Suspensionspolymerisation vom Wasser-in-Öl-Typ einer mehrbasigen Aminosäure (A-1) mit mindestens einer ethylenisch ungesättigten Doppelbindung in einem Molekül und/oder einer mehrbasigen Aminosäure (A-2) ohne ethylenisch ungesättigte Doppelbindung im Molekül und einer Verbindung (B) mit mindestens einer ethylenisch ungesättigten Doppelbindung im Molekül in Gegenwart eines Phosphorsäureester-Tensids der folgenden allgemeinen Formel (1):

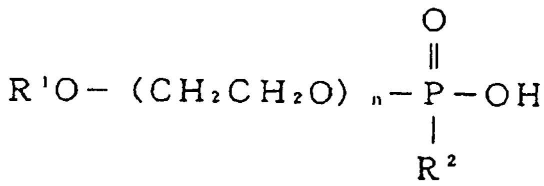

(1)

durchgeführt wird, worin $R^1$ für eine Alkylgruppe oder eine Alkylarylgruppe mit 8 bis 30 Kohlenstoffatomen steht, n eine ganze Zahl von 1 bis 30 bedeutet und $R^2$ eine Hydroxylgruppe oder $R^1O\text{-}(CH_2CH_2O)_n\text{-}$ ist ($R^1$ und n sind wie oben definiert).

8. Absorptionsartikel umfassend einen Absorptionskern enthaltend ein Absorptionsmaterial und ein Fasermaterial, und ein schichtartiges Material, das auf beiden Oberflächen des Absorptionskerns vorgesehen ist, worin das Absorptionsmaterial ein Absorptionsmaterial nach Anspruch 1 ist.

## Revendications

1. Matériau absorbant comprenant une résine absorbante contenant un acide aminé polyacidique comme composant constituant de la résine et ayant une structure dans laquelle les particules principales sont agglomérées, **caractérisé par** l'inclusion de pores ayant un volume de pore total, comme mesuré par un procédé de pénétration de mercure (procédé de porosimétrie par pénétration de mercure), de 0,5 à 5,0 $cm^3/g$.

2. Matériau absorbant selon la revendication 1, dans lequel les pores de la résine absorbante ont une surface de pore totale de 0,1 $m^2/g$ ou plus.

3. Matériau absorbant selon la revendication 1 ou 2, dans lequel la résine absorbante contient un polymère vinylique et un acide aminé polyacidique comme composant constituant de la résine.

4. Matériau absorbant selon la revendication 3, dans lequel la résine absorbante a une structure dans laquelle un acide aminé polyacidique ayant une double liaison à insaturation éthylénique est greffé avec le polymère vinylique.

5. Matériau absorbant selon la revendication 1 ou 2, dans lequel la résine absorbante a une densité apparente de 0,1 à 0,6 g/ml.

6. Matériau absorbant selon la revendication 1 ou 2, dans lequel la résine absorbante est sous la forme de particules ayant un diamètre de particule moyen de 100 à 1000 $\mu m$.

7. Matériau absorbant selon la revendication 1 ou 2, dans lequel la résine absorbante est obtenue en réalisant la polymérisation en suspension en phase inversée de type eau dans l'huile d'un acide aminé polyacidique (A-1) ayant au moins une double liaison à insaturation éthylénique dans une molécule et/ou un acide aminé polyacidique (A-2) n'ayant pas de double liaison à insaturation éthylénique dans une molécule et un composé (B) ayant au moins une double liaison à insaturation éthylénique dans une molécule en présence d'un tensioactif de type ester phosphorique représenté par la formule générale (I) suivante :

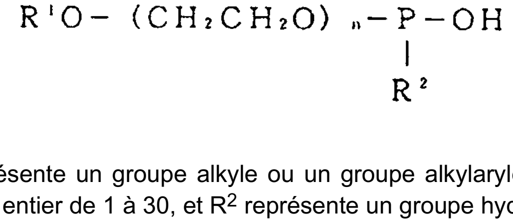

(1)

dans laquelle $R^1$ représente un groupe alkyle ou un groupe alkylaryle ayant de 8 à 30 atomes de carbone, n représente un nombre entier de 1 à 30, et $R^2$ représente un groupe hydroxyle ou $R^1O\text{-}(CH_2CH_2O)_n\text{-}$ ($R^1$ et n sont tels que définis ci-dessus).

**8.** Article absorbant comprenant un noyau absorbant incluant un matériau absorbant et une fibre, et un matériau de type feuille placé sur les deux surfaces du noyau absorbant, dans lequel le matériau absorbant est un matériau absorbant selon la revendication 1.

FIG. 1